# FASCICULE DE BREVET EUROPEEN

(11) **EP 2 576 596 B1**
(45) Date de publication et mention de la délivrance du brevet: **20.01.2016**
(21) Numéro de dépôt: 11727245.0
(22) Date de dépôt: 20.05.2011
(51) Int. Cl.: C07K 7/08, A61K 38/00

(54) **PEPTIDE EN TANT QUE MÉDICAMENT, EN PARTICULIER POUR LE TRAITEMENT DU CANCER**
PEPTID ZUR VERWENDUNG ALS MEDIKAMENT, IM BESONDEREN ZUR BEHANDLUNG VON KREBS
PEPTIDE FOR USE AS A MEDICAMENT, IN PARTICULAR FOR THE TREATMENT OF CANCER

(30) Priorité: 27.05.2010 FR 1054106
(43) Date de publication de la demande: 10.04.2013
(73) Titulaire: Fellous, Esther Suzy Arlette, 75005 Paris (FR); Kalil, Jorge, Sao Paulo SP (BR); Palma, Mario, 13506-765 Rio Claro SP (BR)
(72) Inventeur: Fellous, Esther Suzy Arlette, 75005 Paris (FR); Kalil, Jorge, Sao Paulo SP (BR); Palma, Mario, 13506-765 Rio Claro SP (BR)
(74) Mandataire: Laget, Jean-Loup
(86) Numéro de dépôt international: PCT/FR2011/051150
(87) Numéro de publication internationale: WO 2011/148083

(56) Documents cités:
- WO-A1-00/02581
- DE SOUZA BIBIANA MONSON ET AL.: "Monitoring the positioning of short polycationic peptides in model lipid bilayers by combining hydrogen/deuterium exchange and electrospray ionization mass spectrometry", BIOCHIMICA ET BIOPHYSICA ACTA, vol. 1778, no. 12, 25 septembre 2008 (2008-09-25), pages 2797-2805, XP025658338, ISSN: 0005-2736, DOI: 10.1016/j.bbamem.2008.09.005

## Description

La présente invention se rapporte à une composition pharmaceutique comprenant au moins un peptide particulier pour son application comme médicament dans le traitement du cancer.

Pour traiter le cancer, il existe conventionnellement trois sortes de thérapies : la chirurgie qui vise à retirer la tumeur, la radiothérapie qui vise à détruire la tumeur par des rayons, et les traitements médicaux (chimiothérapie). La chirurgie et la radiothérapie sont des traitements lourds difficiles à supporter pour le malade. En outre, ces trois traitements doivent être le plus souvent associés, car complémentaires. Ils doivent être combinés dans le cadre d'une stratégie programmée.

En ce qui concerne les traitements médicaux, ils ont pour but de traiter non seulement l'organe atteint, mais aussi l'organisme tout entier par l'administration de médicaments anticancéreux. Ainsi, à la différence de la chirurgie ou de la radiothérapie qui ne traitent la tumeur que localement, seuls les traitements médicaux ont une action générale permettant de traiter une tumeur qui a déjà disséminé ou qui est en voie de le faire. Il existe plusieurs types de traitements médicaux : la chimiothérapie cytotoxique et l'hormonothérapie sont les plus classiques ; l'immunothérapie, la thérapie génique et les thérapies non cytotoxiques sont encore du domaine de la recherche.

Le document WO 00/02581 concerne l'utilisation d'un peptide pour la fabrication d'un médicament destiné au traitement ou à la prophylaxie du cancer, le peptide étant constitué notamment par les séquences EARPALLTSRLRFIPK, DGLRPIVNMDYVVGAR, GVPEYGCVVNLR-KTVVNF, ILAKFLHVVL ou ELLRSFFYV.

La publication « monitoring the positioning of short polycationic peptides in model lipid bilayers by combining hydrogen/deuterium exchange and electrospray ionization mass spectrometry » de Bibiana Monson de Souza et Mario Sergio Palma, issu de Biochimica et Biophysica Acta, 2008, pages 2797 à 2805, décrit l'utilisation d'un spectromètre de masse d'ionisation électrospray pour analyser les propriétés d'échange hydrogène/deutérium du peptide Apoica-MP INWLKIAKKVAGML-NH2.

Malgré une recherche approfondie dans ce domaine, il n'existe pas encore à l'heure actuelle un médicament qui soit à la fois efficace dans le traitement de cancers et qui préserve les cellules saines d'un organisme humain ou animal. La chimiothérapie d'aujourd'hui entraîne toujours des effets secondaires d'une gravité plus ou moins grande.

La présente invention a pour but de proposer un nouveau peptide médicament et une nouvelle composition pharmaceutique qui évitent tout ou partie de ces effets secondaires.

A cet effet, l'invention a pour objet un peptide comprenant au moins une séquence choisie parmi : les séquences SEQ. 2, SEQ,3, SEQ. 4, SEQ. 7, SEQ.9, SEQ. 18 et SEQ. 19.

L'invention concerne également un peptide comprenant au moins une séquence choisie parmi : les séquences SEQ. 1, SEQ. 2, SEQ. 3, SEQ. 4, SEQ. 7, SEQ. 9, SEQ. 18 et SEQ. 19, pour une utilisation comme médicament.

De préférence, les peptides de séquences SEQ. 1 à SEQ.25 selon la revendication 3 sont utilisés dans le traitement du cancer.

En particulier, les séquences des peptides agissant en tant que principe actif pour traiter le cancer sont les suivantes (tableau I) :

**Tableau I**

| **Numéro de séquence SEQ (code EZ)** | **Séquences** | **Nombre d'acides aminés** |
|---|---|---|
| 1 (EZ 6) | INWLKIAKKVAGML-NH2, soit | 14 |
| | | |
| 2 (EZ 7) | INWLKIAKKVAGML, soit | 14 |
| 3 (EZ 8) | INWLAIAKKVAGML-NH2, soit | 14 |
| | | |
| 4 (EZ 9) | INWLAIAAKVAGML-NH2, soit | 14 |
| | | |
| 5 (EZ 10) | INWLKIAAAVAGML-NH2, soit | 14 |
| | | |
| 6 (EZ 11) | INWLAIAAAVAGML-NH2, soit | 14 |
| | | |
| 7 (EZ 12) | INWKKIAKKVAGML-NH2, soit | 14 |
| | | |
| 8 (EZ 13) | INWLKIKKKVAGML-NH2, soit | 14 |
| | | |
| 9 (EZ 14) | INWLKIAKKVKGML-NH2, soit | 14 |
| | | |
| 10 (EZ 15) | INWKKIKKKVAGML-NH2, soit | 14 |
| | | |
| 11 (EZ 16) | INWKKIKKKVKGML-NH2, soit | 14 |
| | | |
| 12 (EZ 17) | LKIAKKVAGML-NH2, soit | 11 |
| | | |
| 13 (EZ 18) | LKIAKKVAGML, soit | 11 |
| | | |
| 14 (EZ 19) | KIAKKVAGML-NH2, soit | 10 |
| | | |
| 15 (EZ 20) | KIAKKVAGML, soit | 10 |
| | | |
| 16 (EZ 1) | Biotin-INWLKIAKKVAGML-NH2, soit | 14 |
| | | |
| 17 (EZ 2 | FITC-INWLKIAKKVAGML-NH2, soit | 14 |
| | | |
| 18 (EZ 3 | INWLKIAKKVAGM-NH2, soit | 13 |
| | | |
| 19 (EZ 4) | INWLKIAKKVAG-NH2, soit | 12 |
| | | |
| 20 (EZ 5) | INWLKIAKKVA-NH2, soit | 11 |
| | | |
| 21 (EZ 21) | INALKIAKKVAGML-NH2, soit | 14 |
| | | |
| 22 (EZ 22) | INALKIAKKVAGML, soit | 14 |
| | | |
| 23 (EZ 23) | INKLKIAKKVAGML-NH2, soit | 14 |
| | | |
| 24 (EZ 24) | INKLKIAKKVAGML, soit | 14 |
| | | |
| 25 (EZ 25) | KLKIAKKVAGML-NH2, soit | 12 |
| | | |

Les travaux du demandeur sur ces peptides de SEQ.1 à SEQ.25 ont permis de mettre en évidence, de manière surprenante, que ces peptides présentent une activité cytotoxique contre des cellules cancéreuses tout en n'étant pas ou très faiblement toxiques, pour les cellules de l'hôte à traiter. Les peptides actifs selon l'invention altèrent en effet de manière spécifique les microtubules des cellules cancéreuses.

Constituants d'un des trois réseaux de filaments du cytosquelette, les microtubules sont des polymères complexes et instables dont l'unité de base est le dimère de tubulines α et ß. Ils sont à l'origine de multiples mouvements cellulaires, transport axonal, ou motilité par exemple et surtout ils fournissent le matériau de base pour la constitution du fuseau mitotique. Les microtubules sont des tubes creux de longueur variable et en perpétuelle reformation. Ainsi, en perturbant la dynamique des microtubules des cellules cancéreuses, les peptides selon l'invention agissent également en tant qu'anti-mitotiques empêchant la prolifération des cellules cancéreuses (ralentissement ou arrêt de la mitose des cellules cancéreuses).

Les peptides actifs selon l'invention altéreraient également de manière spécifique les membranes plasmiques des cellules cancéreuses et n'induiraient pas ces mêmes altérations dans les cellules saines de l'organisme hôte. Ceci s'expliquerait par le fait que les peptides actifs, à cause de leur caractère polycationique, agiraient sur les membranes plasmiques des cellules cancéreuses riches en charges négatives.

Par conséquent, les peptides actifs selon l'invention présentent une action sur deux compartiments cellulaires différents : le cytosquelette intracellulaire et la membrane plasmique des cellules cancéreuses.

Les peptides actifs, qui ne comprenant que 10 à 14 acides aminés, présentent aussi l'avantage d'être très faciles à synthétiser.

De préférence, le ou les peptides cités dans le *tableau I,* appliqués pour une utilisation comme médicament pour le traitement du cancer, sont notamment efficaces pour le traitement du cancer du sein, du glioblastome, cancer du cerveau, du col utérin, colorectal, cutanée, endomètre, estomac, foie, gastrointestinale stromale, des hémopathies malaignes (leucémie, myélome multiple, lymphomes (maladie de Hodgkin, lymphone non-hodgkinien), carcinome hépatocellulaire, sarcome de Kaposi, cancer du larynx, mésothéliome, cancer de l'oesophage, de l'oeil, ostéosarcome, cancer de l'ovaire, du pancréas, de la peau (notamment bouche), cancer du poumon, de la prostate, rhabdomyosarcome, cancer du rein, du sein, du testicule, de la thyroïde, des tissus mous, carcinome de la vessie, myélome (cancer osseux) et plasmocytome.

Selon une autre caractéristique de l'invention, les peptides de séquences SEQ.1 à SEQ.25 sont produits par synthèse chimique.

Un autre but de la présente invention concerne une composition pharmaceutique comprenant un des peptides tels que définis ci-dessus.

Avantageusement, la composition se présente sous forme d'une solution, d'une suspension aqueuse, ou à l'état sec sous forme de comprimés nus ou enrobés, comme les pilules, les gélules, les capsules ou les poudres.

Si la composition pharmaceutique est à l'état sec, elle peut être mélangée à un ou plusieurs diluants inertes tels que l'amidon, la cellulose, le saccharose, le lactose ou la silice, etc. Elle peut également comprendre d'autres substances à l'état sec comme un ou plusieurs lubrifiants, tels que le stéarate de magnésium ou le talc, un colorant, un enrobage (dragées) ou un vernis.

Si la composition pharmaceutique selon l'invention est sous fome liquide, elle peut comprendre des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. La composition pharmaceutique peut comprendre d'autres substances liquides autres que les diluants, par exemple des produits mouillants, édulcorants, épaississants, aromatisants ou stabilisants.

De manière préférée, la composition, associée éventuellement à un excipient ou véhicule inerte non toxique pharmaceutiquement acceptable, est caractérisée en ce qu'elle est administrable par voie topique sous forme d'application cutanée, transcutanée ou percutanée ; orale ; parentérale ; nasale ou bronchique.

La composition pharmaceutique pour administration parentérale peut être de préférence une solution aqueuse ou non aqueuse, une suspension ou une émulsion. Comme solvant ou véhicule, on citera l'eau, le propylèneglycol, des huiles végétales, en particulier l'huile d'olive ou l'huile de sesame, des esters organiques injectables, par exemple l'oléate d'éthyle ou d'autres solvants organiques convenables. La composition pharmaceutique peut également contenir des adjuvants, en particulier des agents mouillants, isotonisants, émulsifiants, dispersants et stabilisants.

Pour l'administration par voie topique, les médicaments seront administrés avantageusement sous forme de pommades, crèmes, gels ou de patchs.

En particulier, la composition pharmaceutique est stérile.

La stérilisation peut se faire de plusieurs façons, par exemple par filtration aseptisante, en incorporant à la composition des agents stérilisants, par irradiation ou par chauffage. Les médicmantes peuvent également être préparés sous forme de compositions solides stériles qui peuvent être dissoutes au moment de l'emploi dans de l'eau stérile ou tout autre milieu stérile injectable.

La composition pharmaceutique pourra de plus être utilisée seule ou en association, dans un traitement donné avec d'autres médicaments, ou associée à de la radiothérapie ou de la chirurgie.

L'invention sera mieux comprise, et d'autres buts, détails, caractéristiques et avantages de celle-ci apparaîtront plus clairement au cours de la description suivante d'un mode de réalisation particulier de l'invention, donné uniquement à titre illustratif et non limitatif, en référence aux dessins annexés. Des exemples des essais pharmaceutiques sont en outre proposés afin d'illustrer, mais en aucun cas ils ne sauraient être interprétés comme limitant la portée de l'invention.

Sur ces dessins :

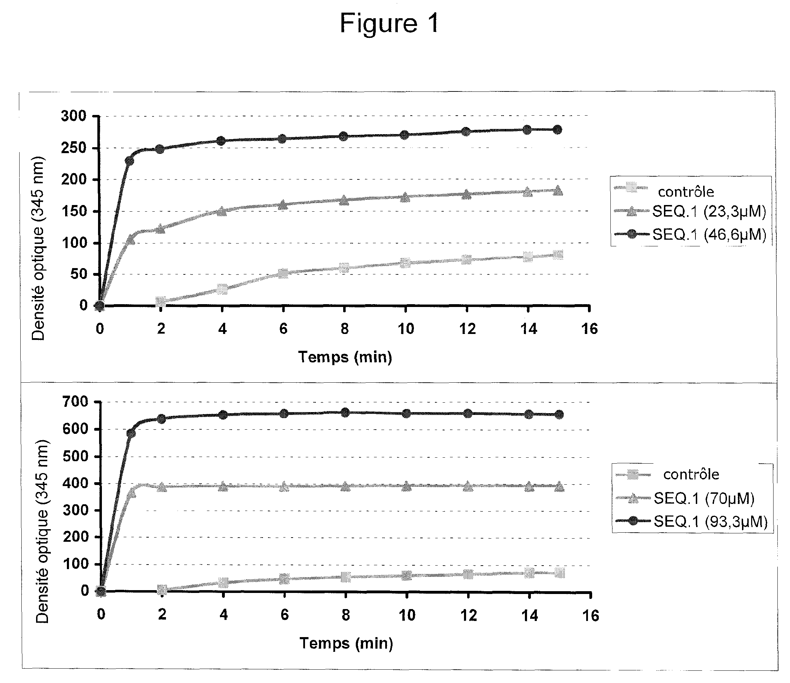

- la figure 1 représente l'effet du peptide de SEQ.1 sur la polymérisation de microtubules d'un cerveau de rat sain à des doses de 23,3 µM, 46,6 µM, 70 µM et 93,3 µM, ainsi que l'effet d'un échantillon témoin n'ayant pas été soumis au peptide de SEQ.1 ; en particulier la figure 1 représente l'évolution de la polymérisation *in vitro* de microtubules de cerveau de rat, quantifiée par des mesures de densité optique à 345 nm de longueur d'onde, en fonction du temps en minutes et en présence ou non de peptide de SEQ.1 ;

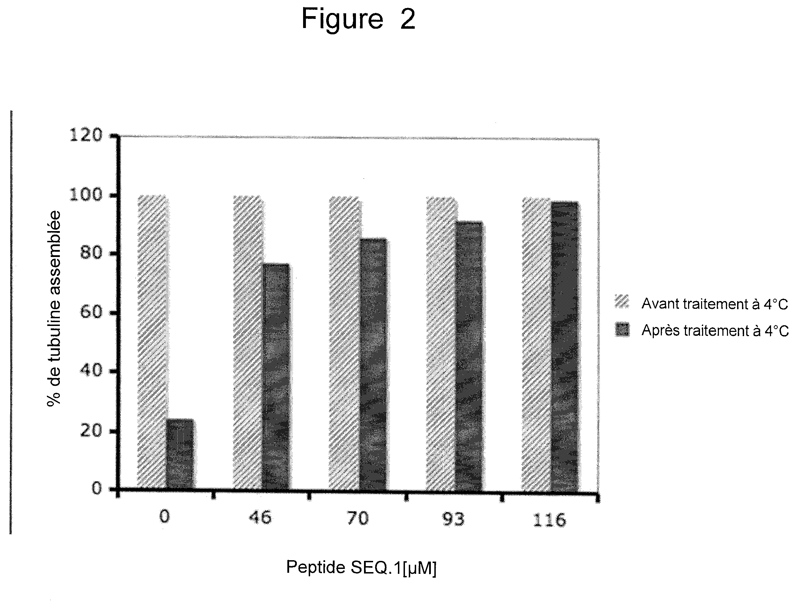

- la figure 2 représente l'effet du peptide de SEQ.1 aux doses de 46,6 µM, 70 µM, 93,3 µM et 116 µM sur la stabilité des microtubules de cerveau de rat sain, évaluée par le pourcentage de dépolymérisation microtubulaire induit par un traitement de 30 min à 4°C des microtubules préalablement assemblés à 37°C pendant 15 min ;

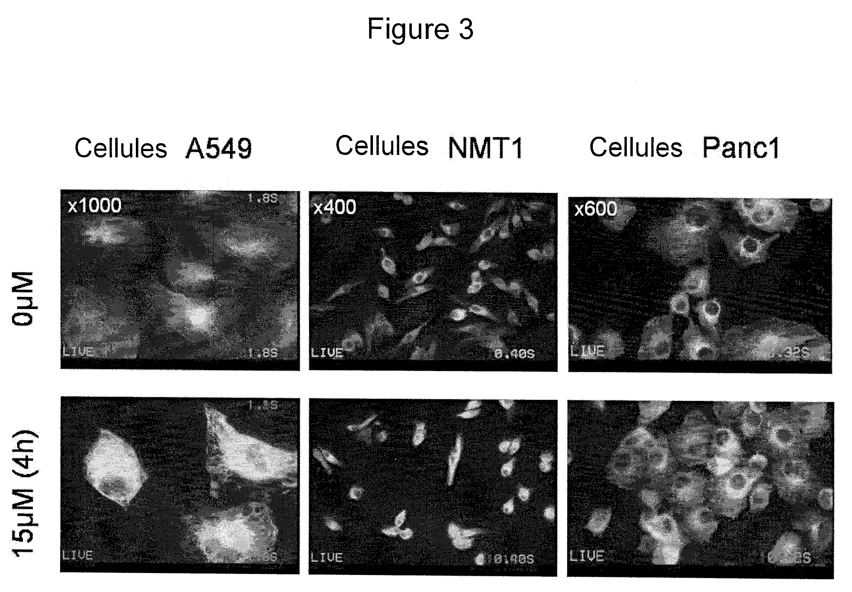

- la figure 3 représente des photos de la lignée cellulaire A549 (cancer du poumon) traitée avec le peptide de SEQ.1 à 15 µM et non traitée avec ce peptide (contrôle à 0 µM), de la lignée cellulaire NMT-1 traitée avec le peptide de SEQ.1 à 15 µM et non traitée avec ce peptide (contrôle) et de la lignée cellulaire PANC-1 (cancer du pancréas) traitée avec le peptide de SEQ.1 à 15 µM et non traitée avec ce peptide (contrôle) ;

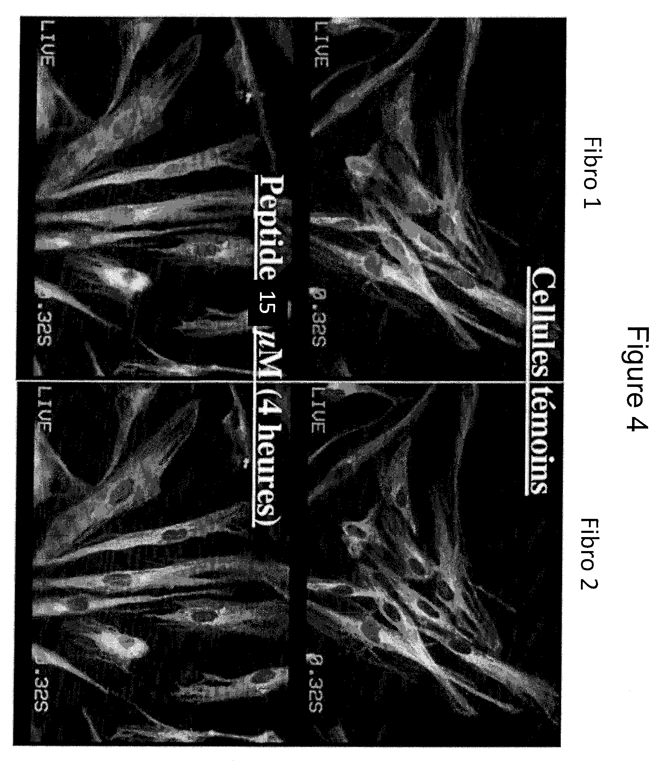

- la figure 4 représente des photos de lignées cellulaires non tumorales, FIBRO 1 et FIBRO 2 (cellules fibroplastiques) traitées avec le peptide de SEQ.1 à 15 µM et non traitées avec ce peptide (contrôle) ;

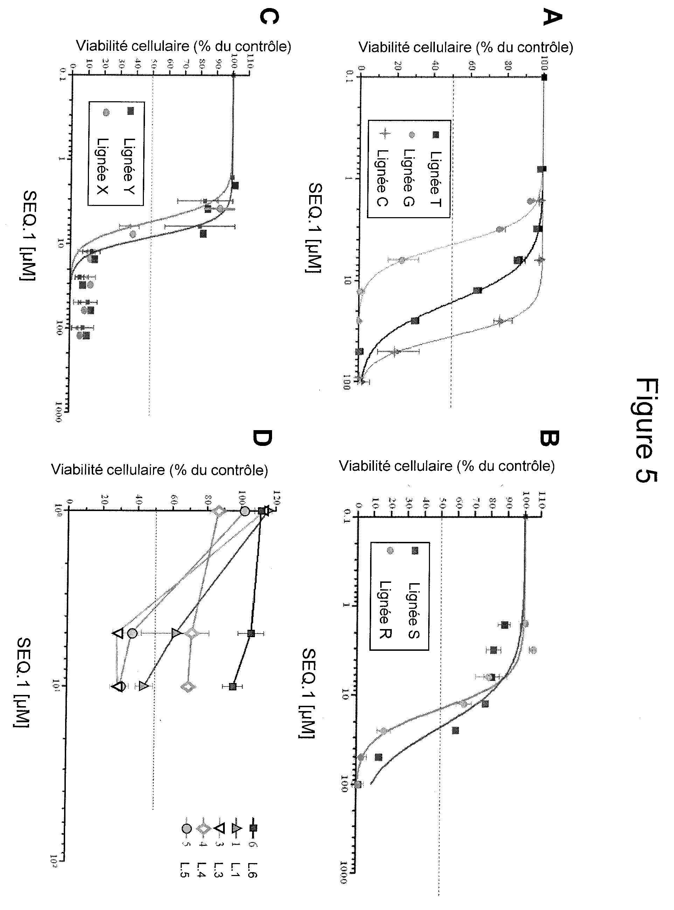

- la figure 5 représente quatre graphiques représentant la viabilité des cellules de différentes lignées : T (lignée de rein Cos); G (lignée de poumon A549); C (lignée de foie HepG2); R (lignée pancréatique PANC1); S (lignée pancréatique MiaPaCa); X (lignée surréalienne H2 95 R) ; Y (lignée de mélanome MNT-1); lignées lymphocytaires leucémiques L1 (HL 60) ; L3 (U 937); L4 (NB 4); L5 (MénoMac6), et une lignée de lymphocytes normaux L6, et ce en fonction de la concentration du peptide de SEQ.1

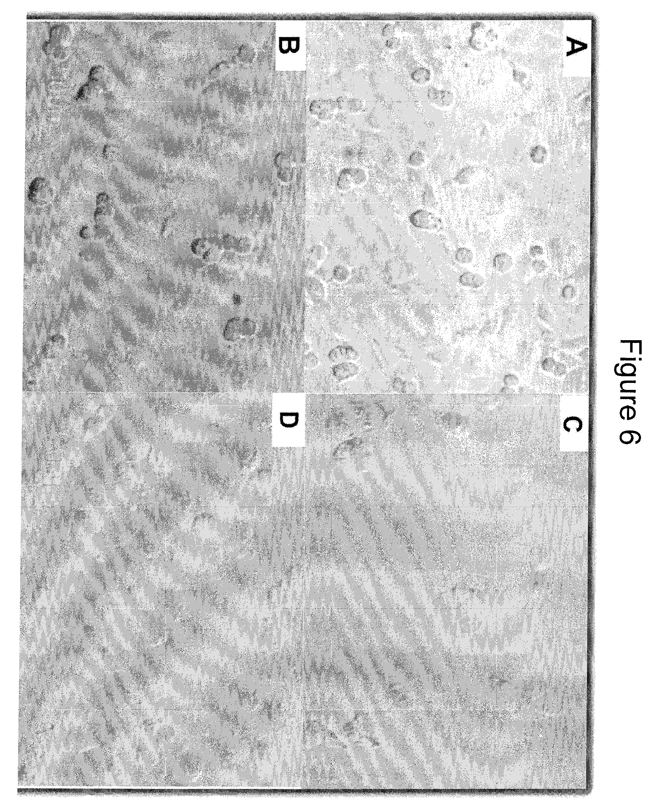

- la figure 6 représente 4 images en contraste de phase de cellules cancéreuses A 549 (cancer du poumon) montrant leur morphologie en l'absence du peptide de SEQ.1 (images témoins A et B) et les altérations de leur morphologie induites par 15 µM du peptide de SEQ.1 (image C prise 4 heures après l'injection du peptide de SEQ.1 et image D prise 2 h après l'injection du peptide de SEQ.1) ; et

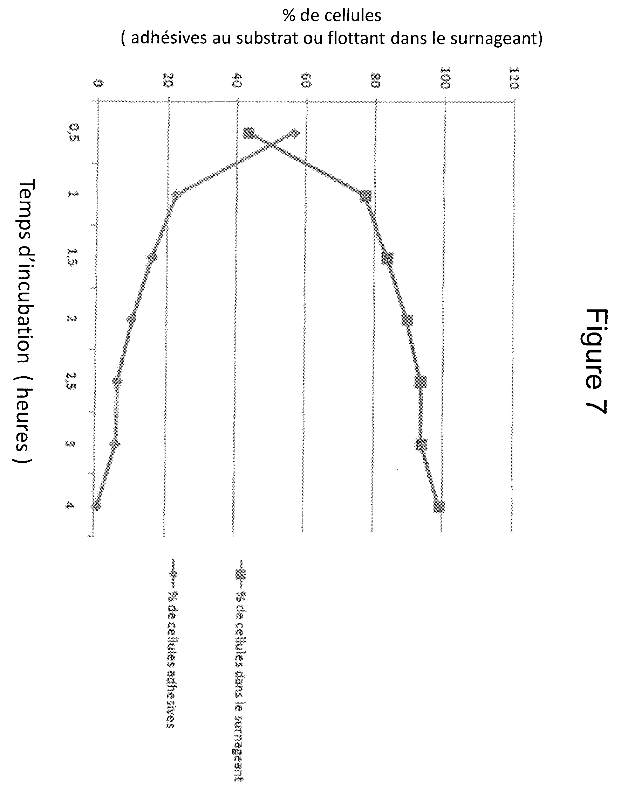

- la figure 7 est un graphique montrant l'effet du peptide de SEQ. 1 (concentration de 15 µM) sur la perte d'adhésivité au substrat des cellules cancéreuses A 549 en fonction du temps.

### A) MATERIELS ET METHODES

### A1) Synthèse des peptides SEQ.1 à SEQ .25 et purification

Les peptides sont préparés par une synthèse sur phase solide fractionnée manuellement en utilisant le N-9-fluorophénylméthoxy-carbonyle avec une résine TGS Novasyn. Les chaînes latérales protègent les groupes incluant t-butyle pour la sérine et t-butoxycarbonyle pour la lysine. Pour préparer les peptides présentant un résidu N-terminal acétylé, après le couplage du dernier résidu d'acide aminé, de l'acide anhydrique a été ajouté aux complexes résine-peptide et maintenu sous agitation pendant 1 heure pour promouvoir l'acétylation.

Le clivage des complexes résine-peptide a été réalisé par traitement avec l'acide trifluoroacétique / 1,2-éthanedithiol / anisole / phénol / eau (82,5 :2,5 :5 :5 :5 par volume) à une concentration de 10 mL.g-1 à température ambiante pendant 2 heures. Une filtration a ensuite été effectuée pour éliminer la résine. Puis, de l'éther éthylique à 4°C a été ajouté, provoquant la précipitation des peptides bruts qui ont été ensuite collectés après une centrifugation à 1 000 g pendant 15 minutes à température ambiante. Les peptides bruts ont ensuite été solubilisés dans de l'eau et chromatographiés sous RP-HPLC en utilisant une colonne semi-préparative (250 mm* 10,0 mm ; 5 µm) C-18 de Shiseido sous une élution isocratique avec 40% (v/v) d'acétonitrile contenant 0,1% (v/v) de TFA à un taux de 2 mL/min. L'élution a été régulée à 215 nm avec un détecteur UV-DAD (détecteur ultraviolet à barettes de diodes) (Shimadzu, modèle SPD-M10A) et chaque fraction éluée a été manuellement collectée dans une fiole en verre de 2 mL. Les séquences des peptides synthétisés (les acétylés comme les non acétylés) ont été déterminées par analyses HPLC et ESI-MS.

### A2) Peptides de SEQ.1 à SEQ.25

Ainsi, après que les peptides ont été synthétisés, purifiés et examinés, des tests ont été effectués pour déterminer leurs activités biologiques. La plupart d'entre eux se sont avérés également présenter une activité antibactérienne.

De plus, il est apparu de manière surprenante et inattendue que les peptides selon l'invention présentaient des propriétés qui altèrent les microtubules de cellules cancéreuses, et notamment le peptide SEQ.1 pour lequel les résultats sont décrits ci-dessous. Ce peptide est un peptide court de 14 acides aminés. Ce peptide présente la séquence d'acides aminés suivante : INWLKIA KKVA GML-NH2 (SEQ.1).

Les peptides de séquence SEQ.2 à SEQ .25 sont des variants ou homologues du peptide de SEQ.1. Ces variants diffèrent du peptide SEQ.1 par un à trois acides aminés (absence ou substitution d'un ou plusieurs acides aminés) et/ou par l'absence du groupe -NH₂ à l'extrémité N-terminal. Ces variants se sont avérés, soit tout aussi efficaces que le peptide de SEQ.1, soit ont montré une efficacité légèrement moindre que le peptide de SEQ.1, mais généralement ils sont plus actifs sur des types de cancers qui répondent un peu moins bien au peptide SEQ.1.

### B) TESTS EXPERIMENTAUX

### B1) Effet du peptide de séquence SEQ.1 sur les propriétés des microtubules purifiées (figure 1).

Des microtubules purifiés de cerveau de rat ont été préparés par un procédé *in vitro* dépendant de la température d'assemblage à 37°C et de désassemblage à 4°C en présence de GTP (guanosine triphosphate) décrit par FELLOUS et al. (1977) « microtubule assembly in vitro, purification of assembly-promoting factors », Eur J Biochem, 15 août 1977, 15 ; 78(1) :167-174.

La polymérisation *in vitro* des microtubules a été suivie par la mesure des changements de turbidité à 345 nm toutes les 30 secondes au cours d'une période d'incubation de 15 min. Les mesures de densité optique ont été effectuées avec un spectrophotomètre UVICON thermostaté à 37°C et équipé d'un chargeur automatique pouvant accueillir 6 cuves.

Tel que représenté sur la figure 1, le peptide de SEQ.1 induit une augmentation importante de la turbidité pendant le processus de la polymérisation de microtubules par rapport à l'échantillon témoin ne comprenant pas le peptide de SEQ.1 et servant de contrôle de la polymérisation de microtubules. L'augmentation d'absorbance observée en présence du peptide de SEQ. 1 va de paire avec l'augmentation de sa concentration et peut atteindre un niveau très élevé, qui ne peut s'expliquer que par la seule augmentation de microtubules normaux polymérisés. Ces données impliquent ainsi la formation de structures de microtubules anormaux ou d'agrégats non organisés de tubuline en présence du peptide de SEQ.1. Par conséquent, la figure 1 démontre que le peptide SEQ.1 a une action directe sur des microtubules purifiées et que par conséquent, les microtubules intracellulaires devraient constituer une cible du peptide. D'ailleurs, cette polymérisation de microtubules anormaux ressemble à la polymérisation de microtubules qui a lieu en présence d'un fort taux d'un anticancéreux déjà connu : le Taxol.

### B2) Action du peptide SEQ.1 : similaire à l'action du Taxol (figure 2)

Un autre argument qui suggère fortement que le peptide de SEQ.1 et le Taxol présentent une certaine similitude dans leur mécanisme d'action est démontrée à la figure. 2.

Le même type d'expériences utilisant la même technologie que pour *B1)* a été réalisé pour évaluer la stabilité des microtubules formés en présence de concentrations croissantes du peptide de SEQ.1. Cette stabilité est indiquée par l'incapacité de ces microtubules à se dépolymériser après un traitement à 4°C pendant 30 min.

En effet, une diminution de capacité des microtubules à se dépolymériser est observée en présence de SEQ.1 lorsqu'ils sont soumis à la température de 4°C pendant 30 minutes. Cette diminution est d'autant plus importante que la concentration de SEQ.1 est élevée. Elle devient totale si la concentration de SEQ.1 est très élevée. Les microtubules formés en présence de concentrations très élevées du peptide de SEQ.1 perdent en effet leur capacité à se dépolymériser lorsqu'ils sont soumis pendant 30 min à une température de 4°C.

Le fait que le peptide de SEQ.1 présente un mécanisme d'action très proche de celui du Taxol est un avantage certain de la présente invention. Il faut également ajouter que les observations du demandeur démontrant l'effet irréversible du peptide de SEQ.1 sur les propriétés microtubulaires lui donnent un avantage majeur par rapport à d'autres peptides anti-microtubulaires comme les dolostatines dont l'effet sur les microtubules est réversible.

Avec des essais au niveau cellulaire, il est confirmé que le peptide de SEQ.1 a un mécanisme d'action similaire à celui du Taxol. Ces essais montrent en effet que le peptide de SEQ.1 augmente de façon anormale et irréversible la concentration intracellulaire de tubuline polymérisée et /ou agrégée induisant ainsi un blocage du processus de mitose.

Cependant le peptide de SEQ.1 ne semble pas se lier sur le même site de la tubuline que le Taxol car plusieurs lignées obtenues à partir de tumeurs du sein résistantes au taxol et qui ont gardé cette propriété de résistance en culture. Ces cellules *in vitro* ont montré une grande sensibilité au peptide de SEQ.1 et à quelques analogues de ce peptide (cf. paragraphe B9).

Une telle observation est majeure car il sera possible d'envisager une thérapie efficace grâce aux peptides SEQ.1 à 25 à des patients qui ne répondent pas ou ne répondent plus à un traitement par le Taxol.

### B3) Effet du peptide de SEQ.1 sur l'altération d'organisation du réseau de microtubules des cellules cancéreuses via un marquage par immunofluorescence des tubulines assemblées (figures 3).

Pour vérifier l'hypothèse que le peptide de SEQ.1 pourrait empêcher la prolifération de cellules cancéreuses en altérant le réseau de microtubules (c'est-à-dire en ayant un mécanisme d'action proche de celui du (« mécanisme taxol-like »), des expériences de marquage par immunofluorescence des structures de microtubules des cellules traitées ou pas par le peptide de SEQ.1 ont été réalisées en employant le procédé décrit ci-dessous.

Des cellules cancéreuses de différentes lignées (A549 : cancer du poumon, NMT1 : mélanome, Panc1 : cancer du pancréas) ont été incubées sur des lames de verre avec différentes concentrations du peptide de SEQ.1 pendant 4 heures à 37° C. Ensuite, les cellules ont été fixées avec du PFA, lavées au PBS et incubées dans un tampon PBS (tampon phosphate salin) contenant 1% de BSA, 0,1% de Triton X 100 pendant 30 minutes à 37°C. Après lavage avec du PBS, les cellules ont été incubées avec un anticorps monoclonal anti-bêta tubuline durant la nuit dans une chambre froide. Ensuite les lames de verre ont été lavées avec du PBS et les cellules ont été incubées avec un anticorps de chèvre antisouris couplé à de l'isothiocyanate de fluorescéine (FITC) pendant 60 minutes à la température ambiante. Après lavage au PBS, les cellules marquées ont été visualisées avec un microscope à fluorescence. La technique de coloration par le dapi a été employée pour marquer les noyaux en bleu.

Tel que représenté sur la figure 3, lorsque les cellules A 549 du cancer du poumon sont traitées par le peptide de SEQ.1, et ce même pendant un court laps de temps, il y a formation de polymères très denses de tubuline qui sont un mélange de microtubules et d'agrégats. Lorsque ces cellules ne sont pas traitées avec le peptide de SEQ.1, le réseau de microtubules est très organisé et non perturbé. La figure 3 montre aussi l'effet du peptide de SEQ.1 sur deux autres variétés de cellules cancéreuses, les cellules de mélanome MNT-1 et les cellules pancréatiques PANC 1. Lorsque ces cellules sont traitées avec le peptide SEQ.1, leur réseau microtubulaire est également altéré comme le montre l'augmentation significative de la fluorescence par rapport à la fluorescence des cellules non traitées. Cependant l'altération microtubulaire observée dans les cellules MNT-1 et PANC 1 traitées est moins importante que dans les cellules A549. Ceci peut s'expliquer par le fait que la période du traitement n'était pas suffisante ou par le fait que les tubulines des lignées MNT-1 et PANC 1 présentent des différences dans leur structure moléculaire par rapport aux tubulines des cellules A549 et seraient ainsi capables d'induire une réduction de leur affinité pour le peptide SEQ.1.

### B4) Activité du peptide de SEQ.1 sur des cellules saines (figure 4)

L'activité spécifique du peptide de SEQ.1 sur les cellules cancéreuses et non sur les cellules saines a été confirmée par les expériences montrées sur la figure 4. Deux variétés de cellules fibroblastiques non tumorigènes : FIBRO 1 et FIBRO 2 ont été mises en contact avec le peptide de SEQ.1 afin de voir si le réseau de microtubules de cellules saines était également perturbé par le peptide. Or, la figure 4 démontre que les fibroblastes humains qui représentent une variété de cellules normales ne répondent pas du tout au peptide de SEQ.1 (pas de modification de densité de la tubuline polymérisée), tout au moins à des concentrations en peptide de SEQ.1 utilisées pour les cellules cancéreuses (15µM). Cette observation représente une avancée importante de la présente invention. Très peu d'agents antitumoraux présentent une très grande différence de cytotoxicité pour les cellules cancéreuses versus les cellules saines de l'organisme hôte (c'est-à-dire très toxique pour les cellules cancéreuses et faiblement toxique pour les cellules saines de l'hôte).

Ce dernier point a deux conséquences importantes : il est en effet très probable que peu d'effets secondaires apparaîtront quand le peptide de SEQ.1 et ses analogues seront employés comme agent thérapeutique chez le mammifère, en particulier chez l'Homme.

De plus, il sera possible d'augmenter de manière significative l'efficacité du peptide de SEQ.1 et de ses analogues en augmentant, soit leur dosage, soit leur temps de traitement.

### B5) Mesure de la prolifération et de la viabilité cellulaires (figures 5)

Les lignées cellulaires suivantes (*tableau II*) ont été analysées pour déterminer et quantifier l'activité du peptide de SEQ.1. Sur le tableau II qui suit, les fibroblastes M et N ne sont pas tumoraux, les lymphocytes L.1, L.3, L.4 et L.5 sont leucémiques alors que les lymphocytes L.6 sont des lymphocytes normaux.

Environ 10 000 cellules ont été ensemencées dans un volume de 0,2 mL par puits dans des microplaques de 96 puits pendant 24 heures avant traitement par le peptide de SEQ.1. Les cellules ont été incubées de 48 à 72 heures à 37°C dans un incubateur CO₂ en présence ou non du peptide de SEQ.1 à différentes concentrations.

Suite à cette incubation, du réactif MTT ou bromure de 3-(4,5 diméthyl-2-yl)-2,5 diphényl tétrazolium (0,5 mg/ml dans un volume de 0,1 ml) a été ajouté à chaque puits et incubé pendant 2 heures à 37°C.

**Tableau II**

| **Type de tissu** | **Lignée cellulaire** | **Code choisi pour la lignée cellulaire** |
|---|---|---|
| Cancer du poumon | A549 | G |
| Cancer du foie | HepG2 | C |
| Glioblastome | U 87 -M 6 | P |
| Cancer du pancréas | MiaPaCa | S |
| Cancer du pancréas | PANC- 1 | R |
| Neuroblastome | SK N F 1 | P |
| Glande surrénale | H2.95 R | X |
| Mélanome | MNT-1 | Y |
| Chorio-épithéliome | JEG | D |
| Chorio-épithéliome | BEWO | E |
| Cancer de la prostate | DU 145 | L |
| Cancer de la prostate | LnCaP | K |
| Cancer du sein | T 47 | B |
| Cancer du colon | HT 29 | F |
| Fibroplastes IVG | FIBRO 1 | M |
| Fibroplastes N 12 | FIBRO 2 | N |
| Lymphocytes Leucémiques | HL 60 | L. 1 |
| Lymphocytes Leucémiques | U 937 | L. 3 |
| Lymphocytes leucémiques | NB 4 | L. 4 |
| Lymphocytes Leucémiques | MenoMac 6 | L. 5 |
| Lymphocytes normaux | | L. 6 |

L'anneau de tétrazolium que contient le MTT est alors réduit par la succinate deshydrogénase des cellules vivantes en formazan. Ensuite, le surnageant a été retiré et 0,1 ml de tampon de lyse (isopropanol contenant 10 % de triton X 100 et 10 % d'HCl 1N) a été ajouté pour dissoudre le précipité bleu-violet de formazan formé. Après quelques minutes, l'absorbance a été déterminée à température ambiante en utilisant un lecteur de microplaque à une longueur d'onde test de 562 nm. Des puits ne comprenant pas de peptide de SEQ.1 ont été utilisés de manière à contrôler la viabilité de cellules de contrôle. Des valeurs d'IC 50 (concentration de peptide de SEQ.1 provoquant 50 % de cytotoxycité sur ces lignées cellulaires) pour chaque variété de cellules ont été évaluées par le rapport de l'absorbance des cellules traitées sur l'absorbance des cellules de contrôle.

Une autre méthode de mesure de la viabilité cellulaire a été utilisée. Cette méthode consiste à effectuer un comptage direct visuel du nombre de cellules, en s'aidant d'une cellule de Malassez, dans diverses conditions de traitement. Cette méthode permet de mieux évaluer les étapes précédent la mort cellulaire (meilleure visibilité au microscope par vision directe de cellules) que par le test MTT.

Tel que cela est représenté sur la figure 5 et le tableau III, le peptide de SEQ.1 empêche la prolifération des cellules tumorales. Plus la dose du peptide de SEQ.1 est augmentée et plus la prolifération de cellules cancéreuses est empêchée. Il a été observé que les doses de peptide SEQ.1 toxiques pour les cellules cancéreuses ne le sont pas pour des cellules normales comme les lymphocytes normaux ou les fibroblastes. D'après le demandeur, le peptide SEQ.1 diminue la prolifération des cellules cancéreuses probablement en altérant la formation des microtubules du fuseau mitotique.

Il a également été observé que la sensibilité des différentes variétés de cellules tumorales varie. *Le tableau III* ci-dessous prouve que la valeur d'IC 50 est très basse pour les cellules tumorales très sensibles, telle que la variété de cellule A549 (cancer du poumon). La valeur d'IC 50 est plus haute pour des variétés de cellules moins sensibles telles que les cellules pancréatiques (variété de cellules PANC 1) ou de variétés de cellules de mélanome humain MNT 1 (variété de cellules DU 145) ou de variétés de cellules de glioblastome (U87- M6). Dans ce dernier cas, la sensibilité dépend du temps d'exposition des cellules de gliobastome avec le peptide de SEQ.1. Cette observation suggère que le peptide de SEQ.1 pourrait présenter une action et une efficacité différente selon le type de cellules cancéreuses. Une modulation de la dose ou de la longueur du traitement peut alors être envisagée pour agir efficacement sur certains types de cancers. L'ensemble des résultats du tableau III montre bien que le peptide de SEQ. 1 a une très large activité antitumorale qui pourrait être plus importante que celle du taxol pour combattre différents types de cancers.

Le tableau III montre aussi la très faible toxicité du peptide SEQ.1 sur différents types de cellules normales comme les lymphocytes ou les fibroblastes.

Ce tableau montre que le peptide de SEQ.1 présente une activité sur un grand panel des cellules cancéreuses (mesure de la viabilité cellulaire).

**Tableau III**

| *Après 48 heures de traitement par le peptide de SEQ.1* | | | | |
|---|---|---|---|---|
| **Type de cellules** | | **Code des lignées cellulaires** | | **IC50** |
| A 549 (Tumeur de poumon humain) | | G | | 4,40 |
| COS (Tumeur du rein de singe) | | T | | 16,48 |
| MiaPaCa (Tumeur du pancréas humain) | | S | | 23,10 |
| PANC 1 (Tumeur du pancréas humain) | | R | | 14,35 |
| H2 95R(Tumeur de la médullosurrénale) | | X | | 5,40 |
| MNT-1 (Mélanome humain) | | Y | | 8,30 |
| U87 M6 (Glioblastome) | | P | | 15,26 |
| HepG2 (Hépatome) | | C | | 35,60 |
| SK-N-F1 (Neuroblastome) | | O | | 2,88 |
| Lignée cellulaire de leucémie aiguë promyélocytaire | | L.1 | | 7,90 |
| Lignée cellulaire de leucémie | | L.3 | | 4,10 |
| Lignée cellulaire de leucémie aiguë promyélocytaire | | L.4 | | 52,40 |
| Lignée cellulaire monocytique | | L.5 | | 4,50 |
| Lymphocytes normaux | | L.6 | | > 10 |
| Fibroblastes | | M | | 17,80 |
| Fibroblastes | | N | | 22 |

| *Après 72 heures de traitement par le peptide de SEQ.1* | | | | |
|---|---|---|---|---|
| **Type de cellules** | **Lignée cellulaire** | **Code des lignées cellulaire** | **IC50** | |
| Glioblastome | U87-M6 | P | 6,00 | |
| | | Fibro 1 et 2/ | > 25 | |

### B6) Effets du peptide de SEQ. 1 sur la structure de la membrane plasmique (figures 6 et 7)

Les effets de SEQ.1 sur la structure et propriétés de la membrane plasmique ont été mis en évidence par des expériences de vision directe des cellules traitées au microscope à contraste de phase et par des expériences de comptage des cellules qui adhèrent et de celles qui ont perdu leur capacité d'adhérer au substrat.

La figure 6 montre qu'en présence du peptide de SEQ.1, le nombre de cellules tumorales A549 a fortement diminué (après 2 et 4 heures de traitement) et qu'en plus, leur contour cellulaire est très altéré par suite des altérations de la membrane plasmique Ceci s'ajoute aux altérations affectant le cytosquelette microtubulaire.

La figure 7 montre en effet que le nombre de cellules A 549, qui conserve leurs propriétés adhesives au substrat, diminue en fonction du temps d'incubation et qu'au contraire, le nombre de cellules A 549 qui flottent dans le surnageant à cause d'une perte d'adhésivité au substrat, augmente en fonction du temps de culture.

Le peptide de SEQ.1, par son caractère polycationique modifie les propriétés des membranes plasmiques des cellules cancéreuses riches en phospholopides de charge négative en déstabilisant la structure de la double couche lipidique de ces cellules. Cette déstabilisation membranaire entraîne des déformations cellulaires soit en induisant des extensions cytoplasmiques soit des déformations du contour cellulaire comme le montre l'expérience illustrée dans la figure 6 où l'on voit dans les cellules traitées un contour cellulaire anormal avec augmentation du volume cellulaire. La déstabilisation des membranes entraïne aussi des changements fonctionnels comme la perte d'adhésion au substrat de culture comme l'indique la figure 7.

Ces altérations membranaires sont responsables de morts cellulaires importantes. Donc, le peptide de SEQ.1 et certains variants exercent un double effet, cytostatique (inhibition des mitoses) et cytotoxique (morts cellulaires). Ce double effet amplifie l'action antitumorale de ces peptides.

Le peptide de SEQ.1 et certains analogues peuvent ne pas altérer seulement les membranes de cellules cancéreuses. Ils peuvent altérer les membranes d'autres types cellulaires comme par exemple des cellules bactériennes ou fungiques.

### B7) Effets des analogues du peptide de séquence SEQ.1

Les analogues ou variants du peptide SEQ.1 ont été testés sur la lignée A549 qui correspond à un cancer du poumon encore très difficile à soigner. Les variants testés sont les suivants : SEQ.3, SEQ.4, SEQ.7, SEQ. 9, SEQ.18, SEQ.19 et SEQ.2.

L'évaluation de l'activité de différents variants testés peut être résumée comme suit :

| | | |
|---|---|---|
| 5 µm <IC < 10 µm | SEQ.3, SEQ.4, SEQ.7, et SEQ.9 | Très bonne activité proche de celle du peptide SEQ.1 |
| IC 50 voisin de 15 µM | SEQ.18, SEQ.19 et SEQ.2 | Activité moyenne |

En conclusion de l'analyse de l'activité des variants du peptide de SEQ 1, il est possible de dire qu'au moins quatre peptides dont présentant une séquence modifiée par rapport à celle de SEQ1, à savoir les peptides de SEQ.3, SEQ.4, SEQ.7, et SEQ.9, ont une activité cytotoxique tout à fait équivalente au peptide SEQ.1 vis à vis des cellules A 549.

Un autre résultat concerne le peptide de SEQ.7. Ce peptide s'est révélé non seulement actif contre les cellules A 549 mais aussi, il présentait une certaine activité contre les cellules de mélanome MNT-1 avec un IC 50 se situant entre 10 et 15 µM et également contre les cellules de cancer du sein T47 avec un IC 50 entre 10 et 15 µM.

En outre, en augamnetant le dosage ou en augmentant le traitement dans le temps des peptides, il sera possible d'améliorer l'efficacité du traitement contre le cancer. Par exemple, en augmentant le dosage de SEQ.1, il sera possible d'obtenir de meilleurs résultats pour traiter des tumeurs moyennement sensibles comme certaines tumeurs pancréatiques PANC-1 ou le glyoblastome.

### B8) Résistance des cellules cancéreuses vis-à-vis des peptides SEQ.1 à 25

Enfin, suivant les indications des expériences préliminaires, le peptide de SEQ.1 et ses analogues présenteraient l'avantage de ne pas induire un processus rapide de résistance.

### B9) Sensibilité de cellules résistantes au taxol aux peptides de séquences SEQ.1 et SEQ.7

Les travaux ont été effectués sur 2 lignées ce cellules, TX 1 et TX 2 dérivées de tumeurs du sein qui ne répondent plus au Taxol. Les deux lignées ont montré une très bonne sensibilité à au moins deux peptides à savoir le peptide de SEQ.1 et le peptide de SEQ.7. Plus précisément les résultats d'inhibition de croissance ont été trouvés déjà significatifs après 24 H. de culture en présence de ces peptides.

Après 48 H. de culture en présence des peptides, les évaluations d'inhibition de croissance sont les suivants :

| | IC50 en µM avec peptide de SEQ. 1 | IC50 en µM avec peptide de SEQ. 7 |
|---|---|---|
| les cellules TX 1 | 5 | 3 |
| les cellules TX 2 | 10 | 7 |

Pour les deux types de cellules résistantes TX 1 et TX 2, les peptides SEQ.1 et de SEQ.7 présentent une très bonne sensibilité puisque l'indice IC 50 évalué s'est situé entre 3 et 7 µM.

Bien que l'invention ait été décrite en liaison avec un mode de réalisation particulier, il est bien évident qu'elle n'y est nullement limitée et qu'elle comprend tous les équivalents techniques des moyens décrits ainsi que leurs combinaisons si celles-ci entrent dans le cadre de l'invention.

## Revendications

1. Peptide comprenant au moins une séquence choisie parmi : les séquences SEQ. 2 , SEQ. 3, SEQ. 4, SEQ. 7, SEQ. 9, SEQ. 18 et SEQ. 19.

2. Peptide comprenant au moins une séquence choisie parmi : les séquences SEQ. 1, SEQ.2, SEQ. 3, SEQ. 4, SEQ. 7, SEQ. 9, SEQ. 18 et SEQ. 19, pour une utilisation comme médicament.

3. Peptide selon la revendication 2, pour utilisation dans le traitement du cancer.

4. Peptide selon la revendication 3, pour utilisation dans le traitement des : glioblastome, cancer du cerveau, du col utérin, colorectal, cutanée, de l'endomètre, de l'estomac, du foie, gastrointestinale stromale, des hémopathies malignes (leucémie), myélome multiple, lymphomes (maladie de Hodgkin, lymphone non-hodgkinien), carcinome hépatocellulaire, sarcome de Kaposi, cancer du larynx, mésothéliome, cancer de l'oesophage, de l'oeil, ostéosarcome, cancer de l'ovaire, du pancréas, de la peau (notamment bouche), cancer du poumon, de la prostate, rhabdomyosarcome, cancer du rein, du sein, du testicule, de la thyroïde, des tissus mous, carcinome de la vessie, myélome (cancer osseux) et plasmocytome.

5. Peptide selon la revendication 1, **caractérisé en ce qu'**il est produit par synthèse.

6. Composition pharmaceutique comprenant un peptide ou un mélange de peptides selon la revendication 2.

7. Composition pharmaceutique selon la revendication 6, **caractérisée en ce qu'**elle se présente sous forme d'une solution, d'une suspension aqueuse, ou à l'état sec sous forme de comprimés nus ou enrobés, comme les pilules, les gélules, les capsules ou les poudres.

8. Composition selon la revendication 6, associée éventuellement à un excipient ou véhicule inerte non toxique pharmaceutiquement acceptable, **caractérisée en ce qu'**elle est administrable par voie topique sous forme d'application cutanée, transcutanée ou percutanée ; orale ; parentérale ; nasale ou bronchique.

## Patentansprüche

1. Peptid, umfassend mindestens eine Sequenz, ausgewählt aus den Sequenzen SEQ. 2, SEQ. 3, SEQ. 4, SEQ. 7, SEQ. 9, SEQ. 18 und SEQ. 19.

2. Peptid, umfassend mindestens eine Sequenz, ausgewählt aus den Sequenzen SEQ. 1, SEQ. 2, SEQ. 3, SEQ. 4, SEQ. 7, SEQ. 9, SEQ. 18 und SEQ. 19 für eine Anwendung als Arzneimittel.

3. Peptid nach Anspruch 2 zur Verwendung in der Krebsbehandlung.

4. Peptid nach Anspruch 3 zur Verwendung für die Behandlung des Glioblastoms, des Krebses des Gehirns, des Gebärmutterhalses, des kolorektalen Krebses, des Krebses der Haut, des Endometriums, des Magens, der Leber, des gastrointestinalen Stromaltumors, der malignen Hämopathien (Leukämie), des multiplen Myeloms, der Lymphome (Morbus Hodgkin, Nicht-Hodgkin-Lymphom), des hepatozellulären Karzinoms, des Kaposi-Sarkoms, des Kehlkopfkrebses, des Mesothelioms, des Krebses der Speiseröhre, der Augen, des Osteosarkoms, des Krebses der Eierstöcke, der Bauchspeicheldrüse, der Haut (vor allem des Mundes), des Krebses der Lungen, der Prostata, des Rhabdomyosarkoms, des Krebses der Nieren, der Brust, der Hoden, der Schilddrüse, der Weichteile, des Harnblasenkarzinoms, des Myeloms (Knochenkrebs) und des Plasmozytoms.

5. Peptid nach Anspruch 1, **dadurch gekennzeichnet, dass** es durch Synthese hergestellt ist.

6. Pharmazeutische Zusammensetzung, die ein Peptid oder ein Peptidgemisch nach Anspruch 2 umfasst.

7. Pharmazeutische Zusammensetzung nach Anspruch 6, **dadurch gekennzeichnet dass** sie in Form einer Lösung, einer wässrigen Suspension oder in trockenem Zustand in Form von einfachen oder umhüllten Tabletten wie Pillen, Gelkapseln, Kapseln oder Pulver vorliegt.

8. Zusammensetzung nach Anspruch 6, eventuell in Kombination mit einem Arzneihilfsstoff oder einem pharmazeutisch akzeptablen nicht toxischen inerten Vehikel, **dadurch gekennzeichnet, dass** sie auf topischem Weg in Form einer Anwendung auf der Haut, transkutan oder perkutan, oral, parenteral, nasal oder bronchial verabreichbar ist.

## Claims

1. A peptide comprising at least one sequence selected from: the sequences SEQ. 2, SEQ. 3, SEQ. 4, SEQ. 7, SEQ. 9, SEQ. 18 and SEQ. 19.

2. A peptide comprising at least one sequence selected from: the sequences SEQ. 1, SEQ. 2, SEQ. 3, SEQ. 4, SEQ. 7, SEQ. 9, SEQ. 18 and SEQ. 19, for use as a drug.

3. The peptide according to claim 2, for use in the treatment of cancer.

4. The peptide according to claim 3, for use in the treatment of: glioblastoma, cancer of the brain, the cervix, colorectal, skin cancer, cancer of the endometer, the stomach, the liver, gastrointestinal stromal cancer, malignant hemopathies (leukemia), multiple myeloma, lymphomas (Hodgkin's disease, non-Hodgkin lymphoma), hepatocellular carcinoma, Kaposi sarcoma, cancer of the larynx, mesothelioma, cancer of the esophagus, the eye, osteosarcoma, cancer of the ovary, of the pancreas, of the skin (notably of the mouth), lung, prostate cancer, rhabdomyosarcoma, kidney, breast, testicle, soft tissue cancer, bladder carcinoma, myeoloma (bone cancer) and plasmacytoma.

5. The peptide according to claim 1, **characterized in that** it is produced by synthesis.

6. A pharmaceutical composition comprising a peptide or a mixture of peptides according to claim 2.

7. The pharmaceutical composition according to claim 6, **characterized in that** it appears as a solution, and aqueous suspension or in the dry condition as exposed or coated tablets, like pills, gelatin capsules, capsules or powders.

8. The composition according to claim 6, optionally associated with a pharmaceutically acceptable non-toxic inert excipient or carrier, **characterized in that** it may be administered via a topical route as a cutaneous, transcutaneous or percutaneous; oral; parenteral; nasal or bronchial application form.


### SEQUENCE LISTING

<110> Arlette, FELLOUS
   Jorge, KALIL
   Mario, PALMA
<120> Composition Pharmaceutique pour le traitement du cancer
<130> F09-6429WO
<150> FR1054106
   <151> 2010-05-27
<160> 25
<170> PatentIn version 3.3
<210> 1
   <211> 14
   <212> PRT
   <213> artificial sequence
<220>
   <223> peptide
<400> 1
   Ile Asn Trp Leu Lys Ile Ala Lys Lys Val Ala Gly Met Leu
   1 5 10
<210> 2
   <211> 14
   <212> PRT
   <213> artificial sequence
<220>
   <223> peptide
<400> 2
   Ile Asn Trp Leu Lys Ile Ala Lys Lys Val Ala Gly Met Leu
   1 5 10
<210> 3
   <211> 14
   <212> PRT
   <213> artificial sequence
<220>
   <223> peptide
<400> 3
   Ile Asn Trp Leu Ala Ile Ala Lys Lys Val Ala Gly Met Leu
   1 5 10
<210> 4
   <211> 14
   <212> PRT
   <213> artificial sequence
<220>
   <223> peptide
<400> 4
   Ile Asn Trp Leu Ala Ile Ala Ala Lys Val Ala Gly Met Leu
   1 5 10
<210> 5
   <211> 14
   <212> PRT
   <213> artificial sequence
<220>
   <223> peptide
<400> 5
   Ile Asn Trp Leu Lys Ile Ala Ala Ala Val Ala Gly Met Leu
   1               5                   10
<210> 6
   <211> 14
   <212> PRT
   <213> artificial sequence
<220>
   <223> peptide
<400> 6
   Ile Asn Trp Leu Ala Ile Ala Ala Ala Val Ala Gly Met Leu
   1               5                   10
<210> 7
   <211> 14
   <212> PRT
   <213> artificial sequence
<220>
   <223> peptide
<400> 7
   Ile Asn Trp Lys Lys Ile Ala Lys Lys Val Ala Gly Met Leu
   1               5                   10
<210> 8
   <211> 14
   <212> PRT
   <213> artificial sequence
<220>
   <223> peptide
<400> 8
   Ile Asn Trp Leu Lys Ile Lys Lys Lys Val Ala Gly Met Leu
   1               5                   10
<210> 9
   <211> 14
   <212> PRT
   <213> artificial sequence
<220>
   <223> peptide
<400> 9
   Ile Asn Trp Leu Lys Ile Ala Lys Lys Val Lys Gly Met Leu
   1               5                   10
<210> 10
   <211> 14
   <212> PRT
   <213> artificial sequence
<220>
   <223> peptide
<400> 10
   Ile Asn Trp Lys Lys Ile Lys Lys Lys Val Ala Gly Met Leu
   1               5                   10
<210> 11
   <211> 14
   <212> PRT
   <213> artificial sequence
<220>
   <223> peptide
<400> 11
   Ile Asn Trp Lys Lys Ile Lys Lys Lys Val Lys Gly Met Leu
   1               5                   10
<210> 12
   <211> 11
   <212> PRT
   <213> artificial sequence
<220>
   <223> peptide
<400> 12
   Leu Lys Ile Ala Lys Lys Val Ala Gly Met Leu
   1               5                   10
<210> 13
   <211> 11
   <212> PRT
   <213> artificial sequence
<220>
   <223> peptide
<400> 13
   Leu Lys Ile Ala Lys Lys Val Ala Gly Met Leu
   1               5                   10
<210> 14
   <211> 10
   <212> PRT
   <213> artificial sequence
<220>
   <223> peptide
<400> 14
   Lys Ile Ala Lys Lys Val Ala Gly Met Leu
   1 5 10
<210> 15
   <211> 10
   <212> PRT
   <213> artificial sequence
<220>
   <223> peptide
<400> 15
   Lys Ile Ala Lys Lys Val Ala Gly Met Leu
   1 5 10
<210> 16
   <211> 14
   <212> PRT
   <213> artificial sequence
<220>
   <223> peptide
<400> 16
   Ile Asn Trp Leu Lys Ile Ala Lys Lys Val Ala Gly Met Leu
   1 5 10
<210> 17
   <211> 18
   <212> PRT
   <213> artificial sequence
<220>
   <223> peptide
<400> 17 Met Leu
<210> 18
   <211> 13
   <212> PRT
   <213> artificial sequence
<220>
   <223> peptide
<400> 18
   Ile Asn Trp Leu Lys Ile Ala Lys Lys Val Ala Gly Met
   1 5 10
<210> 19
   <211> 12
   <212> PRT
   <213> artificial sequence
<220>
   <223> peptide
<400> 19
   Ile Asn Trp Leu Lys Ile Ala Lys Lys Val Ala Gly
   1               5                   10
<210> 20
   <211> 11
   <212> PRT
   <213> artificial sequence
<220>
   <223> peptide
<400> 20
   Ile Asn Trp Leu Lys Ile Ala Lys Lys Val Ala
   1               5                   10
<210> 21
   <211> 14
   <212> PRT
   <213> artificial sequence
<220>
   <223> peptide
<400> 21
   Ile Asn Ala Leu Lys Ile Ala Lys Lys Val Ala Gly Met Leu
   1               5                   10
<210> 22
   <211> 14
   <212> PRT
   <213> artificial sequence
<220>
   <223> peptide
<400> 22
   Ile Asn Ala Leu Lys Ile Ala Lys Lys Val Ala Gly Met Leu
   1               5                   10
<210> 23
   <211> 14
   <212> PRT
   <213> artificial sequence
<220>
   <223> peptide
<400> 23
   Ile Asn Lys Leu Lys Ile Ala Lys Lys Val Ala Gly Met Leu
   1               5                   10
<210> 24
   <211> 14
   <212> PRT
   <213> artificial sequence
<220>
   <223> peptide
<400> 24
   Ile Asn Lys Leu Lys Ile Ala Lys Lys Val Ala Gly Met Leu
   1               5                   10
<210> 25
   <211> 12
   <212> PRT
   <213> artificial sequence
<220>
   <223> peptide
<400> 25
   Lys Leu Lys Ile Ala Lys Lys Val Ala Gly Met Leu
   1               5                   10